# EUROPEAN PATENT APPLICATION

(11) **EP 4 342 447 A1**
(43) Date of publication of application: **27.03.2024**
(21) Application number: 22803530.9
(22) Date of filing: 17.05.2022
(51) Int. Cl.: A61K 8/9789, A61K 36/185, A61Q 19/08, A61P 17/18

(54) **EXTRACT OF IN VITRO CULTURES OF ARISTOTELIA CHILENSIS (MAQUI) WITH ANTIOXIDANT, PROTECTIVE AND SKIN-REPAIRING PROPERTIES**

(30) Priority: 17.05.2021 CL 20211287
(71) Applicant: Rubisco Biotechnology, La Reina, Santiago 7880027 (CL); Fundacion Copec Universidad Católica, 7820436 Macul, Santiago (CL)
(72) Inventor: AQUEA ZEBALLOS, Felipe, Santiago, 7880027 (CL)
(74) Representative: Elzaburu S.L.P.
(86) International application number: PCT/CL2022/050052
(87) International publication number: WO 2022/241580

(57) **Abstract**

Composition comprising a plant extract of maqui cells (*Aristotelia chilensis*) grown in vitro with anti-aging and skin regeneration properties, an anti-wrinkle effect and which improves skin density. The extract is preferably aqueous, but optionally it could be an ethanolic or hydroalcoholic extract. The composition comprises the extract and a carrier. It is shown that the composition helps maintain elastin avoiding the degradation of the structure of the skin, improving elasticity and firmness; and induces the antioxidant machinery of skin cells, protecting it from oxidative stress induced by environmental stressors such as ultraviolet light, blue light or air pollution.

## Description

### BACKGROUND OF THE INVENTION

Plant stem cells are undifferentiated and totipotent cells that are located in meristems and are responsible for regenerating plant organs (Heidstra and Sabatini; Nat Rev Mol Cell Biol. 15:30, 2014). In recent years, these plant cells have been used as raw material for the pharmaceutical and cosmetic industries, which has made it possible to discover a new generation of active compounds for use in anti-aging (Kolewe et al; Mol Pharm. 5:243, 2008). The technology related to the cultivation of plant stem cells allows for the production of active compounds that are completely healthy and offer high purity, quality and effectiveness.

Several companies have ventured into the field of plant cell culture and use different types of plants. These companies claim to have products that protect and rejuvenate by activating different physiological mechanisms in the skin tissue, which finally confers properties for the prevention and treatment of chronological aging, as well as the prevention of aging due to the effect of environmental stress such as UV light and pollution.

For example, the Swiss company Mibelle has developed a technology comprising plant cell lysates encapsulated and stabilized in liposomes. This product corresponds to *PhytoCellTec^{™} Malus Domestica,* which according to *in vitro* and *in vivo* tests carried out by Mibelle indicate that it protects the longevity of skin stem cells, delays the senescence of cells and fights chronic aging. These apple stem cells are rich in epigenetic factors and metabolites, which ensure the longevity of skin cells. In addition, its application delays the aging of hair follicles, suggesting a possible use in anti-aging hair preparations.

Another company working on the development of products made from plant stem cells is the Italian company IRB (Istituto di Ricerche Biotecnologiche) which today is part of the cosmetic supplies provider CRODA. They have developed natural active ingredients from plant stem cells using HTN^{™} (High Tech Nature) technology, an industrial method for *in vitro* culture of plant cells from *Leontopodium alpinum, Syringa vulgaris, Echinacea angustifolia* and *Centella asiatica.* The extracts of these cells are used in various cosmetics available in the market in recognized brands such as Origins, Drunk Elephant, Paula's Choice and Juice Beauty, among others.

Vytrus is another company that uses plant cells for cosmic and dermatological use. For this they mainly use fast-growing native plants from different parts of the world.

Regarding the solution proposed by the inventors, it corresponds to preferably aqueous extracts of maqui obtained from *in vitro* cell cultures and compositions comprising them, for use in cosmetic and dermatological applications.

Maqui or *Aristotelia chilensis (Mol.)* is an ornamental plant, known for its characteristic fruit, an edible black berry with antioxidant properties and medicinal uses, also highly desired by birds.

Maqui, also known as kole6n, clone, maquei and queldrón, is a tree native to the subantarctic forests of Chile and Argentina. It grows mainly in boundaries of forests and beds of water courses, always associated with other species of greater importance. It develops preferably in the humid soils of the central valley, in the foothills of both mountain ranges, ravines or forest margins, from sea level to 2,500 m.a.s.l. The maqui has a great morphological plasticity, appearing as a shrub in the northernmost area of its distribution and as a tree in the south.

Currently, it is known that the fruit of Maqui is high in antioxidant capacity, and there are drinks, powders and capsules with extracts of the fruit. The negative part is that the dark juice fruit stains the skin and clothing. The invention, on the other hand, uses leaf cell extracts of this plant, but not of the fruit, so it does not work with dark compositions that stain the skin.

### Previous art.

As already mentioned, there are numerous compositions of plant extracts for skin regeneration applications and with anti-aging properties.

The same inventor has previously developed plant cell extracts for pharmaceutical or cosmetic use, for the prevention of aging. The patent application CL2018002863A1 presents the use of *Fitzroya cupressoides* cells grown *in vitro* to obtain aqueous extracts with anti-aging properties and skin regeneration. In addition, it shows the use and tests to analyze the potential in cell regeneration and anti-aging properties of such extracts in dermatological applications.

In the patent US9289375B2 of STEMTECH INTERNAT INC. cosmetic compositions made with combinations of natural ingredients for skin care that improve its elasticity and hydration and act in the reduction of wrinkles, in addition to preventing aging due to age and environmental conditions. The main ingredient would be a blue-green algae, in addition to aloe, cocoa, vanilla, maqui, among other components, where the plants or their extracts would be used.

For its part, the patent US8828458B2 of MORARIU MARIUS refers to a topical composition containing maqui anthocyanins or a maqui extract, presenting maqui as a powerful anti-aging ingredient, where the problem to be solved is to provide an active compound that does not oxidize. The described composition uses the fruit alone or in combination with maqui leaves, to prevent or treat damage to the skin. In addition to maqui, the composition could contain as additional ingredients collagen and elastin enhancing agents, antiglycation agents, myocontraction, antiedema, antioxidants, etc.

There are developments such as the one shown in the publication WO2017113032A1 of the UNIVERSITY OF SANTIAGO DE CHILE that addresses an antioxidant and antibacterial composition obtained from an in vitro culture of A. *chilensis,* in a Murashige-Skoog (MS) culture medium, where the hydroalcoholic extract obtained would have application in the cosmetic industry or others. Although it is indicated that a natural antioxidant has a preventive function of aging, it does not mention an effect on the processes of skin regeneration or prevention of signs of aging.

In summary, the present invention achieves a composition comprising a standardized extract obtained from cell culture *Aristotelia chilensis* or maqui, which is not anticipated in the previous art. The extract and/or composition allow its use as an active ingredient in the cosmetic industry for its advantageous properties as an anti-aging agent, which stimulates skin regeneration, with anti-wrinkle effects, which help maintain the structure of the skin and protects it from oxidative stress.

### BRIEF DESCRIPTION OF THE FIGURES

Figure 1. Relative viability of HACAT epidermis cells subjected to basal medium without serum with 2% water (C-a), medium with SFB (C-b), hydrogen peroxide 0.1% (C(-)), and extracts of Maqui undiluted M-1 (2% v/v) and diluted 1:2 M-2 (1% v/v), 1:5 M-5 (0.4% v/v) and 1:10 M-10 (0.2% v/v).
Figure 2. Relative viability of human fibroblast cells subjected to serum-free basal medium with 2% water (C-a), medium with SFB (C-b), hydrogen peroxide 0.1% (C(-)), and undiluted Maqui extracts M-1 (2% v/v) and diluted 1:2 M-2 (1% v/v), 1:5 M-5 (0.4% v/v) and 1:10 M-10 (0.2% v/v).
Figure 3. Scratch test at 24 hours of incubation with culture medium supplemented with water (negative control), and extracts of the invention in concentrations of 1.0 and 0.4% v/v of maqui cell extract.
Figure 4. Scratch test at 48 hours of incubation with culture medium supplemented with water (negative control), and extracts of the invention in concentrations of 1.0 and 0.4% v/v of maqui cell extract.
Figure 5. Evaluation of the expression of antioxidant marker genes in epidermal cells (HACAT). Results are shown for SuperOxide Dismutase 1 (SOD1), SuperOxide Dismutase 2 (SOD2), Xeroderma pigmentosum group C protein (XPS) and glutathione S-transferase pi 1 (GSTP1) genes.
Figure 6. Effect of the action of blue light on the viability of human epidermis cells (keratinocytes) and protective role of maqui extract in the decrease of viability caused by blue light.
Figure 7. Graph of the relative expression of the anti-wrinkle marker genes: elastin and aquoporin 3. It is seen that exposure to the extract of the invention strongly stimulates the expression of both genes evaluated.
Figure 8. Graphical representation of the total glutathione content with respect to the reference value. Where cells treated with the extract of the invention increase their glutathione content.

### DETAILED DESCRIPTION OF THE INVENTION

The invention relates to a composition comprising a plant extract of maqui cells (*Aristotelia chilensis*) with anti-aging and skin regeneration properties, anti-wrinkle effects and improvement of skin density. The extract is preferably aqueous, but optionally it could be an ethanolic or hydroalcoholic extract. The composition comprises the extract and a carrier. In addition, the composition helps maintain elastin preventing the degradation of the skin structure, improving elasticity and firmness; and induces the antioxidant machinery of skin cells, protecting it from oxidative stress induced by environmental stressors such as ultraviolet light, blue light or air pollution.

Aqueous, ethanolic or hydroalcoholic extracts of maqui cells (*Aristotelia chilensis*) are obtained from cells grown *in vitro.* The extract is preferably aqueous.

In one embodiment of the invention, maqui cells correspond to stem cells.

The plant extract of maqui cells comprises as major compounds:
- total polyphenols of the order of 100-140 µg/ml gallic acid equivalent,
- flavonoids of the order of 2 to 12 µg/ml quercetin equivalents, and
- flavonols of the order of 30 to 50 µg/ml quercetin equivalent.

The composition is formulated for pharmaceutical or cosmetic use for skin care, and the carrier is at least one excipient for skin care, such as water, glycerin, propylene glycol, Carbopol, polyethylene glycol, pentylene glycol, triethanolamine, mineral oils, natural oils, silicones, dimethicones, or cyclosilicones, for example.

Where the composition comprises formulation excipients and maqui cell extract a final concentration of between 0.0005% w/v to 10% w/v.

The invention also provides the process for making the extract, where fresh *in vitro* cultured maqui cells are collected and dried between 30-40°C for 24 to 60 hours. For example, in an oven at 37°C for 48h, until the water is completely removed. The process includes the following steps:
a. Mixing *in vitro* cultured maqui cells with the solvent, either water or ethanol or a mixture of both, gradually until a concentration of between 0.05% w/v to 15% w/v by dry weight is reached;
b. Incubating the mixture at a temperature between 10 to 40°C with stirring from 100 to 250 rpm for a period of between 1 hour to 5 days; and
c. Separating the liquid phase from the solids, which constitutes the extract,
d. Combining the extract with a carrier.

In a preferred embodiment of the invention, in step b), the concentration of the suspension is between 0.1% w/v to 5% w/v (dry weight with respect to the solvent) of water or ethanol or a mixture of both, for example, 50% ethanol.

In a preferred embodiment of the invention, the maceration or incubation of stage c) is performed between 1 to 3 days, more preferably between 2 to 3 days. More preferably, the incubation is carried out for 24 hours at 25°C with constant agitation and in darkness.

In a preferred embodiment of the invention, in step (d), separation can be performed by centrifugation or filtration. By centrifugation, samples are subjected 10,000 to 20,000 rpm for 10 to 60 minutes, for example, to 14,000 rpm for 20 minutes. For filtration, gauze and paper filters are used, such as Whatman No. 2 filter paper.

Optionally, the extract is sterilized by filtration using, for example, 0.22um filters and stored cold at 4°C until subsequent use.

The extract of the invention has demonstrated its safe use in dermatological applications, being harmless to skin cells. Moreover, the extract shows that it does not promote the development of melanoma cells, confirming that it does not promote the proliferation of malignant cells in the skin. As shown in the examples, the extract of the invention, and the compositions derived from it, exhibit antioxidant activity, protecting the skin against stress and oxidative damage produced by the environment, including ultraviolet light, blue light and air pollution.

The extracts of the invention promote cell regeneration, which allows for affirming that they have anti-wrinkle action, which is seen in the *scratch test* evaluations, where the area of regeneration of groove in monolayer using HACAT cells is evaluated.

The extract of the invention also favors the production of collagen, so that it helps maintain and prevent the degradation of the structure of the skin. Thus the cosmetic or pharmaceutical formulation for skin care of the invention has anti-aging and anti-wrinkle properties and improves skin density, improves the elasticity and firmness of the skin, maintains the structure of collagen and elastin preventing its degradation, and as already indicated, protects the skin from oxidative stress induced by environmental stressors such as ultraviolet light, blue light or air pollution.

All these beneficial properties, which make the extract of the invention an ideal component for skin care compositions will be clearer in the light of the attached examples.

### EXAMPLES

### Example 1: Induction and proliferation of maqui cells in vitro

Plant material from plants grown in the field or seedlings germinated *in vitro* was collected. The initial tissue was adult plant leaf.

The material was sterilized and cultured in Petri dishes with MS (Murashige & Skoog) medium, supplemented with auxin (2,4-D) and cytokinin (BAP) at concentrations of 3 and 1 mg/l respectively and 8 g/l agar.

The plates were grown in darkness at 22°C. After 90 days, it was possible to observe the appearance of cell masses which were cultivated separately to multiply the plant biomass.

Subcultures were performed every 30 days in the same medium. After 6 months, those lines that maintained the proliferation capacity were selected and those that stopped growing were eliminated. For subsequent scaling in bioreactors, those cell lines that grew in liquid medium were selected. To do this, 10 g of maqui cells were dissolved in 100 ml of liquid medium, left stirring in a flask at 100 rpm. They were cultured for 30 days and collected in a graduated test tube. Subsequently, the sedimented cell volume (SCV) is quantified after 30 minutes. Those lines that presented at least 5% SCV were selected.

The selected cell lines correspond to plant stem cells, which have as their main characteristics friable cells (which disintegrate into cell suspensions) and whose growth is unlimited (they do not stop growing if the culture medium is constantly renewed). Maqui stem cells are used for the preparation of extracts used in the following examples.

### Example 2: Preparation of a maqui cell extract according to the invention

2.A The cells were extracted from the plates and dried in an oven at 37°C for 48h 5g in dry weight of maqui cells were weighed and transferred to a flask, 500 ml of water were gradually added, obtaining a suspension at 1% weight/volume.

The mixture was then incubated with stirring at 180 rpm, at room temperature, for 24 hours in darkness.

It was then kept at room temperature at rest, the contents were transferred to 50mL Falcon tubes and centrifuged for 20 minutes at 14000rpm. The precipitated solid tissue was discarded, rescuing the supernatant, thus obtaining the extract of the invention.

Additionally, the extract obtained was filtered through a 0.22 µm membrane for sterilization and stored between 4°C and 20°C.

The plant extract obtained was analyzed obtaining the following profile of major compounds: 120 µg/ml gallic acid equivalent in the case of total polyphenols, 6 µg/ml quercetin equivalent in the case of flavonoids and 38 µg/ml quercetin equivalent for flavonols.

2.B In a second embodiment, cell masses were harvested directly from the petri dish or from cell suspensions grown in liquid medium.

The biomass of maqui cells was dried in an oven at 40°C for 24h.

Subsequently, the dry biomass was ground in a mortar and a 5% w/v solution was prepared using water as a solvent.

Subsequently, the mixture was incubated with stirring at 180 rpm, at 22°C, for 24 hours in darkness. The solution was separated by filtration to remove the solid waste and the supernatant was collected. The contents were transferred to Falcon tubes of 50mL and centrifuged for 20 minutes at 14000rpm to remove solid particles, thus obtaining the extract of the invention.

Additionally, the extract obtained was filtered through a 0.22 µm membrane for sterilization and stored between 4°C and 20°C.

The characterization of the extracts obtained in 2 B is shown in example 2.C

2.C In order to characterize the phytochemical composition of the extracts prepared in example 2.B, spectrophotometric measurements were made.

The measurement of total polyphenols in the extract of maqui cells was performed by the Folin-Cicolteu method. To do this, 10 µl of sample/white/standard were added, 10 µl of Folin-Cicolteu reagent in 50 µl of water, waited 5 minutes and added 30 µl of Na₂CO₃ (7.5% in water) and incubated at RT protected from light 1 hour and measured absorbance at 700 nm. Gallic acid in methanol was used as standard.

For the measurement of flavonoids, 20 µl of sample/white/standard and 20 µl of AlCl₃ (2% in methanol) and 160 µ! of water were added. RT was incubated protected from light for 1 hour and absorbance was measured at 430 nm. Quercetin in methanol was used as standard.

For the measurement of the vanillin index, 20 µl of sample/white/standard, 120 µl of Vanillin 4% in methanol and 60 µl of HCl were added. RT protected from light were incubated for 1 hour and absorbance measured at 500 nm. Catechin in methanol was used as standard. Measurements were made on 2 independently prepared extracts (M1 and M2).

To measure the antioxidant power of maqui cell extract, the TROLOX protocol was used. For this, the QuantiChrom Antioxidant Assay Kit (BioAssay Systems) was used. 20 µ! sample/blank/standard and 100 µl *working reagent* per well were used in duplicate. It was incubated for 10 minutes at room temperature (RT) and absorbance measured at 570 nm. The results of the measurements are presented in Table 1

**Table 1**

| | **Polyphenols** | **Flavonoids** | **Vanillin Index** | **Antioxidant power** |
|---|---|---|---|---|
| **Extract** | µg/ml | µg/ml | µg/ml | µM Trolox |
| M1 | 145.53 | 22.49 | 27.20 | 101.20 |
| M2 | 170.35 | 23.41 | 26.16 | 143.21 |

### Example 3: Evaluation of the effect of maqui cell extract on the viability of human skin cells

To evaluate the effect of the application of an extract of maqui cells of the invention on human skin cells, cultures of keratinocytes and fibroblasts were used.

6000 Hacat cells were seeded in culture medium with 15% SFB per well and 3,000 fibroblasts in culture medium for fibroblasts with 2% fetal bovine serum (FBS) per well. After 24 hours the medium was discarded, washed with PBS 1X and added basal medium without serum with 2% water (C-a), medium with FBS (C-b) and hydrogen peroxide 0.1% (C(-)) in 4 wells each for each cell type and Maqui extracts of the invention obtained in example 2.B (M1 and M2) each undiluted (2% v/v) and diluted 1:2 (1% v/v), 1:5 (0.4% v/v) and 1:10 (0.2% v/v) with water in 4 wells for each condition and cell type.

At 24 hours, a photograph of each condition was taken, the medium was discarded, washed with PBS 1X and 50 µl of basal medium, 50 µl of XTT and 1 µ! of PMS per well were added. It was left incubating for 2 hours and the absorbance of each well was measured at 460 nm (reference 650 nm) in the Tecan Sunrise equipment. The data was processed in an Excel spreadsheet and plotted with the GraphPad Prism 6 program.

First, it is established that there were no apparent changes in the morphology of the cells, both Hacat and Fibroblasts with any of the extracts of the invention in any dilution.

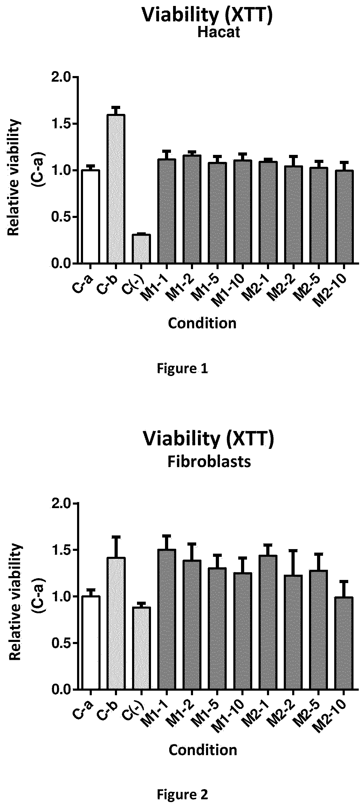

The extracts do not show a significant change in the viability of HACAT epidermis cells (Figure 1). On the other hand, the application of maqui cell extracts of the invention significantly increases the viability of human fibroblast cells when concentrations between 2% v/v and 0.4% v/v are used, as shown in Figure 2.

### Example4: Evaluation of the anti-wrinkle properties of a maqui cell extract

The evaluation of the anti-wrinkle and repairing activity of the skin was performed using the scratch technique. To do this, 35,000 Hacat cells per well were seeded in full medium (15% FBS) in a 96-well dish. At 24 hours, a wound was made with a pipette tip (p100) to each well, washed 2 times with PBS and added basal medium without serum with 2% water (C-a) and half with 15% FBS (C-b) as controls in 8 wells each and Maqui cell extracts diluted 1:2 (1% v/v) and 1: 5 in water (0.4% v/v) in 8 wells each. Photographs were taken in 4X magnification to each well at 0, 24 and 48 hours and wound closure was quantified by Image J. The data was processed in an Excel spreadsheet and plotted with the GraphPad Prism 6 program.

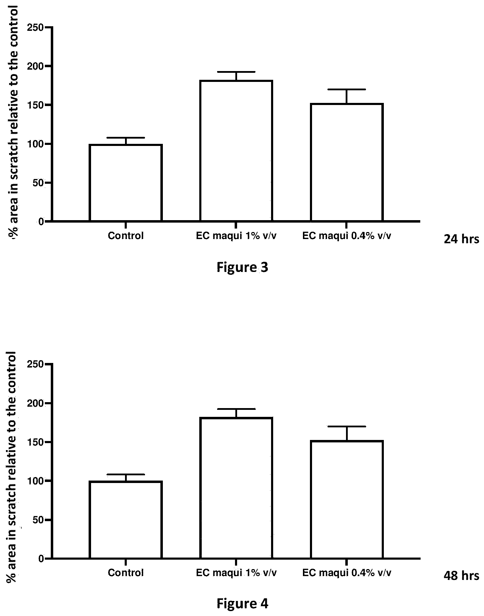

The results indicate that an extract of maqui cells at 1% v/v and 0.4% v/v significantly accelerate the regeneration of a wound in human epidermis (See Figures 3 and 4).

### Example 5: Evaluate the expression of antioxidant marker genes of human epidermis cells in response to maqui cell extract treatments

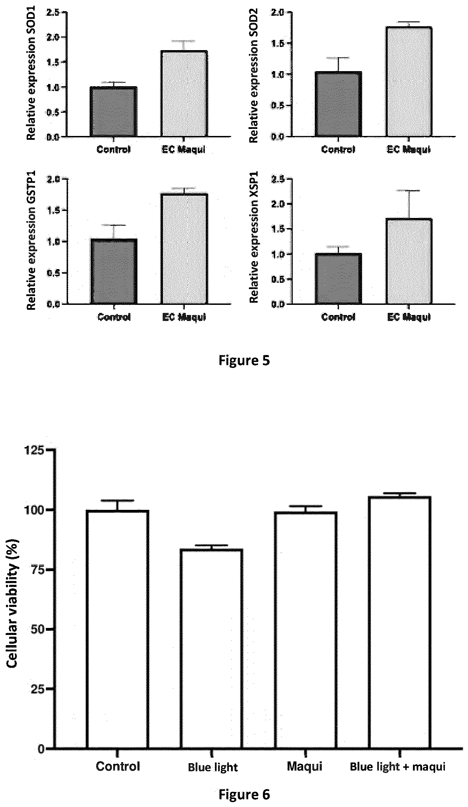

To evaluate the expression of marker genes of the antioxidant machinery in human epidermis cells treated with maqui cell extract of the invention, the following protocol was used:
100,000 keratinocyte cells are seeded per well, in a 6-well plate. HACAT cells are treated with maqui cell extracts at 1% v/v in basal medium for 24h. After finishing the treatments with plant extracts, the culture medium is removed from all wells, and 500µL of TRIzol reagent is added. Subsequently, the total mRNA is extracted according to the manufacturer's instructions. Once the total mRNA was extracted, it was quantified by absorbance. 1µg of each total RNA was taken and treated with 1µL of DNAsa RQ1, to eliminate possible contamination of the samples with cellular DNA, according to the manufacturer's instructions. From DNA-free RNA, 1µg was added to each reverse transcription reaction with the SuperScript II enzyme, according to the manufacturer's instructions. The cDNAs generated were diluted 1/10 in MilliQ water, and 2µL of each cDNA was added to the PCR reaction, prepared with the Brilliant II SYBR Green QPCR Mastermix kit, in a final volume of 20µL of reaction, with each oligonucleotide at a concentration of 0.2mM final. The data was analyzed according to the methodology of ΔΔCt, regarding the expression of the GAPDH gene in the same samples. The genes evaluated were SuperOxide Dismutase 1 (SOD1), SuperOxide Dismutase 2 (SOD2), Xeroderma pigmentosum group C protein (XPS) and glutathione S-transferase pi 1 (GSTP1). The expression of the 4 marker genes of the antioxidant machinery increases significantly in human epidermis cells, as shown in Figure 5.

### Example 6: Protection of a maqui cell extract from damage caused by blue light in epidermal cells

Blue light whose wavelength is between approximately 380 nm and 475 nm is now considered an environmental pollutant that damages human skin. The main sources that emit blue light are computer screens, cell phones and televisions. Blue light triggers oxidative stress in skin cells, which causes a decrease in cell viability. We evaluated whether maqui cell extract is able to protect skin cells from damage caused by blue light. To do this, 30000 human keratinocyte cells are seeded in 96-well plate. The day after planting, the complete DMEM medium is removed, washed with PBS1X, and the cells are treated with a 1% v/v concentration of maqui cell extracts diluted in basal DMEM. Subsequently, the cells are exposed to blue light (blue LED tubes with a maximum emission spectrum in ~460nm), inside the cell incubator at 37°C and 5% CO₂, with the plates with medium and the plate with its lid at a distance of 15cm from the emission source, for a time of 4 hours. After the maximum exposure time is over, 50uL of XTT (1mg/mL) and 1µL of PMS are applied to each well and incubated at 37°C for 60 minutes. Subsequently, the absorbance of the metabolic product of XTT at 460nm is measured, and with this the viability of the cells is obtained. As a result, it is obtained that blue light significantly reduces the viability of human epidermis cells but that treatment with maqui cell extract protects keratinocytes from damage caused by blue light, as shown in Figure 6.

### Example 7: Evaluation of the expression of anti-wrinkle marker genes in human epidermis cells in response to treatments with maqui cell extract of the invention

To evaluate the expression of anti-wrinkle marker genes in human epidermis cells treated with maqui cell extract of the invention, the following protocol was used:
100,000 keratinocyte cells are seeded per well, in a 6-well plate. HACAT cells are treated with maqui cell extracts at 1% v/v in basal medium for 24h. After finishing the treatments with plant extracts, the culture medium is removed from all wells, and 500µL of TRIzol reagent is added. Subsequently, the total mRNA is extracted according to the manufacturer's instructions. Once the total mRNA was extracted, it was quantified by absorbance. 1µg of each total RNA was taken and treated with 1µL of DNAsa RQ1, to eliminate possible contamination of the samples with cellular DNA, according to the manufacturer's instructions. From DNA-free RNA, 1µg was added to each reverse transcription reaction with the SuperScript II enzyme, according to the manufacturer's instructions. The cDNAs generated were diluted 1/10 in MilliQ water, and 2µL of each cDNA was added to the PCR reaction, prepared with the Brilliant II SYBR Green QPCR Mastermix kit, in a final volume of 20µL of reaction, with each oligonucleotide at a concentration of 0.2mM final. The data was analyzed according to the methodology of ΔΔCt, regarding the expression of the GAPDH gene in the same samples. The genes evaluated were ELASTIN and AQUOPORIN 3.

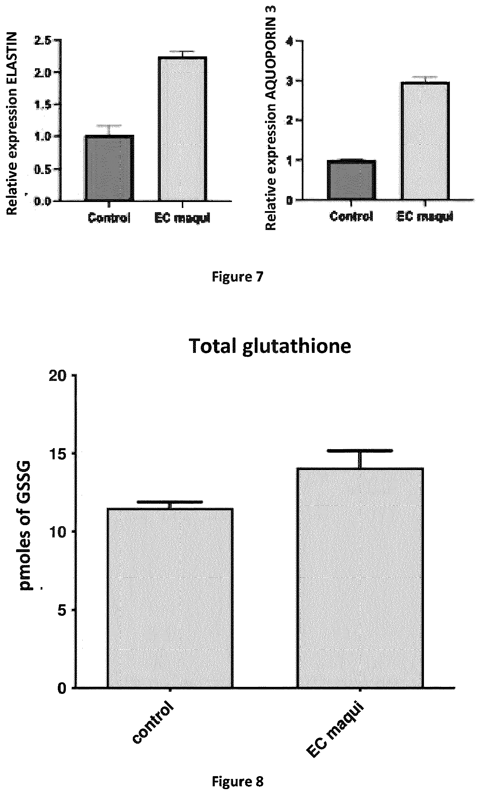

The expression of the 2 anti-wrinkle marker genes evaluated increases significantly in human epidermis cells, treated with the extract of the invention (EC maqui) with respect to the control as shown in Figure 7.

Elastin levels often decrease with skin aging, resulting in, among other effects, wrinkle formation, loss of tensile strength, increased fragility and poor wound healing. AQP3 or aquoporin 3 is the most abundant aquaglyceroporin in the skin and is responsible for transporting water and glycerol in the epidermis. Therefore, AQP3 is a key protein in the maintenance of epidermal hydration and keratinocyte differentiation. Therefore, the positive result observed in the regeneration of epidermis cells is due to the increase in transcripts of elastin and aquoporin 3, which in living tissue results in a decrease in wrinkles and better hydration of the skin, giving a rejuvenated appearance.

### Example 8: Quantification of glutathione content in epidermis cells treated with maqui cell extract of the invention.

To evaluate whether the extract of the invention decreases oxidative stress within epidermis cells, the concentration of glutathione within the treated cells was evaluated. For the expert in the technique it is known that the amount of glutathione present in a cell or tissue is positively correlated with viability and healthy activity. Low glutathione levels thus correlate with oxidative stress and cell death.

300,000 Hacat cells per well were seeded in a 6-well plate with complete medium (15% fetal bovine serum). At 24 hours, each well was washed with PBS 1X and basal medium was added with 2% water and maqui cell extract 1% v/v. At 24 hours it was washed with PBS 1X, the cells of each condition were tripsinized, centrifuged at 300 rcf at 4°C for 10 minutes, the supernatant was thrown away and 1 ml of cold PBS 1X was added and recentrifuged at 300 rcf at 4°C for 10 minutes, the supernatant was discarded and the cells were resuspended with 500 µl of metaphosphoric acid 5% w/v, the supernatant of each condition was transferred to a 1.5 ml tube and stored at -80°C for later use.

For quantification, a standard curve of 100, 50, 25 and 12.5 pmol of glutathione was used and each sample was diluted 1:10, 1:20 and 1:40 with buffer supplied in the kit and 50 µl of each standard, sample and white was loaded in triplicate, 150 µl of mix was added for the reaction containing glutathione reductase per well and the absorbance was measured at 405 nm every 1 minute for 10 minutes.

The average of each point was taken and each curve of the standards, white and sample was plotted by linear regression. The slope of the target was subtracted from the slopes of the standards and samples and the glutathione concentration of the standards vs. absorbance was plotted. Based on this new curve, the amount of glutathione in each sample was quantified.

The results show that treatment with maqui cell extract of the invention significantly induces the content of total glutathione in human keratinocytes. Which allows for asserting that the extract of the invention decreases oxidative stress and stimulates the health of skin cells.

## Claims

1. A composition with regenerative, antioxidant, protective and repairing properties of the skin, **CHARACTERIZED in that** it includes aqueous, ethanolic or hydroalcoholic extracts of maqui cells (*Aristotelia chilensis*) grown *in vitro* and a carrier.

2. The composition according to claim 1, **CHARACTERIZED in that** the maqui cells correspond to stem cells.

3. The composition according to claim 1, **CHARACTERIZED in that** the majority compounds which comprise the extract correspond to total polyphenols of the order of 100-140 mg/ml gallic acid equivalent, flavonoids of the order of 2 to 12mg/ml quercetin equivalents, and flavonols of the order of 30 to 50mg/ml quercetin equivalent.

4. The composition according to claim 3, **CHARACTERIZED in that** the extract corresponds to an aqueous extract.

5. The composition according to claim 4, **CHARACTERIZED in that** the composition is formulated for pharmaceutical or cosmetic use for skin care.

6. The composition according to claim 5, **CHARACTERIZED in that** the carrier is at least one excipient for skin care.

7. The composition according to claim 6, **CHARACTERIZED in that** it comprises formulation excipients and maqui cell extract in a final concentration of between 0.0005% w/v to 10% w/v.

8. Process for obtaining a composition comprising extracts of maqui cells (*Aristotelia chilensis*) grown *in vitro,* according to claim 1, **CHARACTERIZED in that** it comprises the following steps:
a. Mixing the maqui cells cultured *in vitro* with the solvent little by little until reaching a concentration between 0.05% w/v to 15% w/v by dry weight with respect to the solvent;
b. Incubating the mixture at a temperature between 10 to 40°C with stirring from 100 to 250 rpm for a period of between 1 hour to 5 days; and
c. Separating the liquid phase, which constitutes the extract,
d. Combining the extract with a carrier,
wherein the solvent is selected from water, ethanol or a mixture of both.

9. Process according to claim 8, **CHARACTERIZED in that** the maqui cells are previously dried between 30-40°C for 24 to 60 hours.

10. Process according to claim 8, **CHARACTERIZED in that** in step a) the concentration of the suspension is between 0.1% w/v to 5% w/v.

11. Process according to claim 8, **CHARACTERIZED in that** in step b) incubation is between 1 to 3 days.

12. Process according to claim 8, **CHARACTERIZED in that** in step c) separation can be performed by centrifugation or filtration.

13. Use of a composition according to any of claims 1 to 7, **CHARACTERIZED in that** it serves to develop formulations for pharmaceutical or cosmetic use for skin care.

14. Use according to claim 13, **CHARACTERIZED in that** the formulation has anti-aging and anti-wrinkle effects and improves skin density.

15. Use according to claim 13, **CHARACTERIZED in that** the formulation has an effect of improvement in the elasticity and firmness of the skin, maintains the structure of collagen and elastin preventing its degradation.

16. Use according to claim 13, **CHARACTERIZED in that** the formulation protects the skin from oxidative stress induced by environmental stressors such as ultraviolet light, blue light or air pollution.
